# FASCICULE DE BREVET EUROPEEN

(11) **EP 3 040 105 B1**
(45) Date de publication et mention de la délivrance du brevet: **13.12.2017**
(21) Numéro de dépôt: 16150124.2
(22) Date de dépôt: 05.01.2016
(51) Int. Cl.: A61K 8/49, A61Q 19/00, A61K 8/97

(54) **UTILISATION COSMETIQUE D'UN EXTRAIT DE BAIES DE ROSA CANINA PARTICULIER COMME ACTIF ANTI-SEBORRHEE**
KOSMETISCHE ANWENDUNG EINES HAGEBUTTENEXTRAKTS ALS MITTEL GEGEN SEBORRHÖ
COSMETIC USE OF A SPECIFIC EXTRACT OF ROSA CANINA BERRIES AS AN ANTI-SEBORRHEIC ACTIVE AGENT

(30) Priorité: 05.01.2015 FR 1550016
(43) Date de publication de la demande: 06.07.2016
(73) Titulaire: Societe Industrielle Limousine d'Application Biologique, 19130 Objat (FR)
(72) Inventeur: PAUFIQUE, Jean, 19130 OBJAT (FR)
(74) Mandataire: Aquinov

(56) Documents cités:
- DATABASE GNPD [Online] MINTEL; mars 2013 (2013-03), Oberon Cosmetic: "Yeco Solution Anti Greasy Black Head", XP002750557, Database accession no. 2011968
- DATABASE EPODOC [Online] EUROPEAN PATENT OFFICE, THE HAGUE, NL; 25 août 2005 (2005-08-25), Gagnidze Teimuraz: XP002750749, Database accession no. GE-AU2003001131-U & GE U20 051 193 Y (GAGNIDZE TEIMURAZ) 25 août 2005 (2005-08-25)
- ROMAN IOANA ET AL: "Bioactive compounds and antioxidant activity of Rosa canina L. biotypes from spontaneous flora of Transylvania.", CHEMISTRY CENTRAL JOURNAL, vol. 7, no. 1, 73, 23 avril 2013 (2013-04-23), pages 1-10, XP002750558, ISSN: 1752-153X, DOI: 10.1186/1752-153X-7-73
- DATABASE GNPD [Online] MINTEL; janvier 2014 (2014-01), Amorepacific: "Oil Control Pact", XP002747512, Database accession no. 2286691

## Description

La présente invention se rapporte à l'utilisation cosmétique d'un extrait particulier de *Rosa canina* pour lutter contre la sécrétion excessive de sébum et les manifestations associées.

A la fois inconfortable et inesthétique, la peau grasse se manifeste sous la forme d'une peau luisante, sans éclat, rugueuse et déshydratée. Fréquemment accompagnée de pores dilatés, elle favorise également le développement d'imperfections.

En plus des désagréments cosmétiques qu'elle génère, la peau grasse peut également occasionner une gêne dans la vie quotidienne, tant chez les adolescents que chez les adultes. En outre, comme elle touche l'image et l'estime de soi, la peau grasse est aussi susceptible d'engendrer des répercussions psychosociales importantes.

Pour répondre à ces problématiques, il existe déjà plusieurs principes actifs et compositions anti-séborrhée sur le marché. Néanmoins, ces produits ne sont bien souvent pas suffisamment efficaces et n'agissent pas sur l'ensemble des phénomènes à l'origine de la sécrétion excessive de sébum.

En effet, la peau grasse est favorisée par de nombreux facteurs hormonaux, environnementaux et psychologiques. Il s'agit d'un dérèglement des glandes des follicules sébacés, qui se traduit par une sécrétion exagérée de sébum, ou hyper-séborrhée, et marquée principalement dans la zone médiane du visage.

Les glandes sébacées sont situées dans le derme moyen, la plupart sont annexées à un poil, formant ainsi l'unité pilosébacée. Le sébum produit est excrété par le canal folliculaire qui relie la glande à la surface cutanée et dont l'orifice forme un pore.

La quantité de sébum délivrée à la surface de la peau est relative au volume de la glande sébacée. Sa taille est inversement proportionnelle à celle du poil qui lui est annexé. Ainsi trois types de follicules pilosébacés sont distingués sur le visage :
- Le follicule terminal qui comporte un cheveu ou un poil et dont la glande sébacée est petite,
- Le follicule velu qui comporte un duvet associé à glande volumineuse,
- Le follicule sébacé contenant un poil quasiment inexistant et une glande sébacée énorme, multilobée qui s'évacue par un large canal.

La quantité et la distribution de ces follicules va déterminer le type de peau, une prédominance de follicules sébacés favorise la peau grasse.

Le sébum est une graisse fluide dont la fonction principale est de lubrifier les cheveux pour en conserver la souplesse et de protéger la peau en contribuant à la formation de la barrière cutanée. Sa composition est complexe et comprend notamment des triglycérides, des cires, du squalène et du cholestérol.

Le sébum est produit par les sébocytes, cellules épithéliales présentes au niveau des glandes à différents stades de différenciation. En périphérie de la glande, les sébocytes prolifèrent puis se différencient tout en migrant vers le centre de la glande. Au cours de ce processus de maturation, les sébocytes perdent leur activité mitotique et accumulent de larges vacuoles lipidiques. Les sébocytes au stade de différenciation terminale sont gorgés de sébum puis se désintègrent et libèrent leur contenu dans le canal qui relie la glande à la surface de la peau.

Ce programme de différenciation continu est sous le contrôle de médiateurs paracrines, endocrines et neuronaux variés agissant par l'intermédiaire de nombreux récepteurs exprimés par les sébocytes.

L'objectif de la présente invention est de proposer l'utilisation d'un principe actif spécifique qui permet de réduire la différenciation des sébocytes humains, de freiner la sécrétion de sébum et de réduire la surface des pores, pour limiter la brillance et les imperfections de la peau et lutter contre le phénomène de peau grasse.

Pour y répondre, l'invention propose d'utiliser comme actif anti-séborrhée, un extrait hydroglycolique de baies sans akènes de *Rosa canina* contenant des composés polyphénoliques.

L'espèce *Rosa canina* est originaire d'une vaste région, incluant toute l'Europe, de la Méditerranée à la Scandinavie, l'Afrique du Nord, l'Asie mineure, la région du Caucase et de l'Asie centrale et le sous-continent indien. En plus de sa présence dans les régions citées plus haut, la plante est naturalisée dans d'autres régions, notamment en Amérique du Nord et du Sud, et en Océanie.

Rosa canina ou églantier est un arbrisseau buissonnant et grimpant. Les feuilles alternes sont pennées, composées de folioles elliptiques acuminés; les fleurs solitaires ou disposées en corymbes, ont 5 sépales de couleur rose clair ou blanchâtres et sont connues sous le nom d'églantine. Le pseudo-fruit, ou baie, vert, puis jaune, puis rouge, persiste tout l'hiver sur les branches. Le pseudo-fruit est constitué par un réceptacle floral devenu charnu, surmonté par le reste des sépales, de forme ovoïde, généralement de 1 à 2 cm de longueur, et de 0,5 à 1,5 cm de largeur, de couleur rouge orangé à rouge brun à maturité, il renferme les véritables fruits, soit 10 à 20 akènes durs, anguleux, poilus, dénommés par erreur « graines ».

Rosa canina est une espèce végétale bien connue dans ses utilisations alimentaires et médicinales. Les baies d'églantier sont employées comme ingrédient dans certains compléments alimentaires.

Par ailleurs, de nombreuses lignes de produits cosmétiques commercialisent des produits contenant des extraits de *Rosa canina.* Ils utilisent généralement l'huile d'églantier ou un extrait méthanolique ou au CO2 supercritique. Leur composition est très particulière. Ainsi, l'Extrait CO-2 d'Eglantier est riche en acides gras poly-insaturés : 20 - 35 % d'acide alpha-linoléique (oméga-3), 46 - 59 % acide linoléique (oméga-6), 12 - 22 % et d'acide oléique (oméga-9). D'autres constituants actifs sont l'acide palmitique et d'acide stéarique, des caroténoïdes (vitamine A) et de la vitamine E. L'huile d'églantier a une forte teneur en acides gras insaturés essentiels, à savoir les acides oléique (15-20%), linoléique (44-50%) et linolénique (30-35%).

Des principes actifs cosmétiques à base de *Rosa canina* ont donc déjà été proposées, mais aucun ne présente l'efficacité surprenante de l'extrait selon l'invention sur la peau grasse et les manifestations associées, et aucun n'est un extrait hydroglycolique de baies sans akènes de *Rosa canina* contenant des composés polyphénoliques. En outre, contrairement aux actifs à base de *Rosa canina* existants sous forme d'huile essentielle, l'extrait selon l'invention ne présente pas de problèmes de sensibilisation ou d'irritation cutanée et est absent de tout danger toxicologique.

L'invention a donc pour objet l'utilisation cosmétique comme actif anti-séborrhée d'un extrait hydroglycolique de baies sans akènes de *Rosa canina* contenant des composés polyphénoliques, en particulier pour lutter contre la sécrétion excessive de sébum et les manifestations cutanées associées.

Avantageusement l'utilisation sur la peau de l'extrait selon l'invention permet de la purifier, de la matifier et de la débarasser de ses imperfections. L'invention permet ainsi d'améliorer le confort des peaux caucasiennes et asiatiques en particulier.

L'invention a donc également pour objet un procédé cosmétique de soin de la peau pour lutter contre la sécrétion excessive de sébum et les manifestations cutanées associées, qui consiste à appliquer sur la peau une composition cosmétique comprenant un extrait hydroglycolique de baies sans akènes de *Rosa canina* contenant des composés polyphénoliques

D'autres caractéristiques et avantages ressortiront de la description en détails de l'invention qui va suivre.

L'invention a donc pour objet l'utilisation cosmétique comme actif anti-séborrhée d'un extrait hydroglycolique de baies sans akènes de *Rosa canina* contenant des composés polyphénoliques, en particulier pour lutter contre la sécrétion excessive de sébum et les manifestations cutanées associées.

Par « actif » ou « principe actif » au sens de l'invention, on entend un ensemble de plusieurs molécules extraites de baies de *Rosa canina* présentant un effet sur les cellules de la peau.

Par « actif anti-séborrhée » ou « principe actif anti-séborrhée » au sens de l'invention, on entend un actif qui s'il est appliqué sur la peau, présente un effet sur la sécrétion excessive de sébum et les manifestations cutanées associées.

Par « extrait » au sens de l'invention, on entend un ensemble de plusieurs molécules extraites de baies de *Rosa canina.*

Par « extrait hydroglycolique » au sens de l'invention, on entend un extrait obtenu par extraction hydroglycolique c'est-à-dire une extraction faisant intervenir de l'eau et un glycol.

Par « baies de *Rosa canina* » au sens de l'invention, on entend le pseudo-fruit de *Rosa canina,* les baies utilisées selon l'invention étant des baies sans akènes.

Par « composés polyphénoliques » au sens de l'invention, on entend la famille de molécules organiques largement présente dans le règne végétal, caractérisées par la présence de plusieurs groupements phénoliques associés en structures plus ou moins complexes généralement de haut poids moléculaire. Les composés polyphénoliques sont communément subdivisés en phénols simples, acides-phénols (dérivés de l'acide benzoïque ou cinnamique) et coumarines, en naphtoquinones, en stilbénoïdes (deux cycles C6 liés par 2C), en flavonoïdes, isoflavonoïdes et anthocyanes et en formes polymérisées : lignanes, lignines, tanins condensés.

L'actif utile selon l'invention est donc un extrait hydroglycolique de baies sans akènes de *Rosa canina.* Il comprend des composés phénoliques, très préférentiellement notamment des composés polyphénoliques de type flavonoïdes et hydroxybenzoïques. En particulier l'extrait utile selon l'invention comprend des composés hydroxybenzoïques choisis parmi les dérivés de l'acide gallique et de l'acide protocatéchique, et des flavonoïdes choisis parmi la catéchine et ses dérivés et les dérivés de la taxifoline.

Préférentiellement les composés polyphénoliques de type flavonoïdes et hydroxybenzoïques représentent au moins 40% en poids des composés polyphénoliques présents dans l'extrait selon l'invention.

L'actif peut contenir d'autres types de molécules, notamment des sucres.

Selon un mode de réalisation adapté, l'extrait utile selon l'invention se présente sous forme liquide et présente au moins une des caractéristiques suivantes :
- Un taux de matières sèches compris entre 1 et 10g/l, préférentiellement entre 4,5 et 6,5g/l,
- Une teneur en polyphénols totaux comprise entre 5 et 150 mg/l, préférentiellement entre 10 et 20mg/l, soit entre 0.15% et 0.44% de composés polyphénoliques par rapport aux matières sèches,
- Un pH compris entre 3.0 et 4.0.

Le taux de matière sèche peut être mesuré par passage à l'étuve à 105°C en présence de sable d'un échantillon de poids initial donné jusqu'à obtention d'un poids constant.

La teneur en polyphénols totaux peut être mesurée par dosage spectrophotométrique. Les composés phénoliques forment en présence de ferricyanure de potassium et de chlorure de fer des complexes colorés qui peuvent être dosés par spectrophotométrie à 715nm. L'intensité de cette coloration est proportionnelle à la quantité de composés phénoliques présents dans l'échantillon. Les lectures sont réalisés à partir d'une gamme étalon d'acide gallique allant de 10 à 80 mg/L.

La caractérisation des composés phénoliques peut être réalisée par chromatographie, notamment par CLHP/UV (chromatographie liquide haute performance / UV).

L'extrait selon l'invention est obtenu par un procédé d'extraction à l'aide d'eau et de glycol. En particulier l'extrait est obtenu à l'aide d'un solvant constitué par un mélange d'eau et de butylène glycol.

Il peut être obtenu par un procédé comprenant les étapes suivantes :
o Solubilisation de poudre de baies de *Rosa canina* dans un mélange d'eau et de butylène glycol, préférentiellement à raison de 90% de butylène glycol,
o Séparation des phases soluble et insoluble et élimination de la phase insoluble,
o Clarification par filtration de la phase soluble,
o Décoloration,
o Filtration et filtration stérilisante.

Les paramètres des différentes étapes doivent être ajustés afin d'obtenir des principes actifs présentant les caractéristiques de l'invention.

Ces étapes sont usuelles dans le domaine des extractions d'actifs à partir de plantes ou de levures et l'homme du métier est à même d'en ajuster les paramètres réactionnels sur la base de ses connaissances générales.

Les baies de *Rosa canina* sont des baies dépourvues d'akènes, ceux-ci étant notamment potentiellement neurotoxiques par voie orale. De même, les poils hérissés qui recouvrent le réceptacle des baies, ont été éliminés avant de réduire les baies en poudre, car ils constituent le « poil à gratter » et sont très irritants pour la peau et les muqueuses.

L'extrait selon l'invention est préférentiellement utilisé dans une composition, cette composition comprenant un milieu cosmétiquement acceptable. Il s'agit de compositions dans différentes formes galéniques, adaptées à l'administration par voie topique cutanée.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant au moins 0,5% d'extrait de baies de *Rosa canina* selon la présente invention, préférentiellement entre 0,5 et 10%.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les filtres organiques,
- les filtres inorganiques,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic Ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour le soin de la peau humaine. En particulier, l'invention vise un procédé cosmétique de soin de la peau pour lutter contre la sécrétion excessive de sébum et les manifestations cutanées associées. Le procédé consiste à appliquer sur la peau une composition cosmétique comprenant un extrait hydroglycolique de baies sans akènes de *Rosa canina* contenant des composés polyphénoliques, préférentiellement au moins une fois par jour.

En effet l'extrait selon l'invention est particulièrement utile comme actif cosmétique anti-séborrhée, en particulier :
- pour lutter contre la sécrétion excessive de sébum et les manifestations cutanées associées et/ou.
- pour diminuer la sécrétion de sébum, et/ou
- pour réduire la surface des pores de la peau, et/ou
- pour limiter la brillance et les imperfections de la peau liées à la sécrétion de sébum.

C'est notamment la présence de composés polyphénoliques, en particulier de type hydroxybenzoïques et flavonoïdes, qui confère son efficacité à l'extrait.

Le sébum est produit au niveau des glandes par les sébocytes qui se désagrègent à maturité pour libérer les lipides. La glande sébacée mature est ainsi divisée en 3 zones comportant chacune des sébocytes à différents stades de différenciation marqués par des événements cellulaires et moléculaires précis :
- la zone périphérique (40% de la glande) est composée de petites cellules aplaties ou cubiques en prolifération et en contact avec la lame basale. Elles expriment notamment le Ki67 et la kératine 7.
- la zone de maturation, représentant également 40% de la glande, contient des sébocytes en différenciation. Ils perdent leur activité mitotique et accumulent progressivement des lipides sébacés au sein de gouttelettes ; les cellules s'arrondissent et leur volume augmente de façon importante. Cette étape est sous le contrôle d'un facteur majeur, le PPAR-γ (Peroxysome Proliferator-Activated Receptor-γ). Les sébocytes en différenciation expriment également les enzymes impliquées dans la synthèse des lipides (SCD-1, FAR2, DGAT, FATP-4, FADS2) ainsi que dans le métabolisme des androgènes.
- la zone de nécrose (20% de la glande) où les sébocytes en phase terminale de différenciation sont matures et emplis de sébum. Ils se désagrègent à l'approche du canal ; le sébum est relargué à la suite de la lyse et de la dégradation des sébocytes, processus appelé sécrétion holocrine. Les sébocytes en phase terminale de différenciation expriment notamment la FAS (Fatty Acid Synthase) et la Mucin 1.

Le processus de différenciation est d'environ 21 à 25 jours tandis que le temps de transit du sébum dans le canal folliculaire est estimé à 14 heures. Les sébocytes se renouvellent de façon continue, à partir d'une population de cellules souches sébocytaires présente dans le follicule pilosébacé, sous le contrôle de signalisations variées (Wnt/β-catenin, BMP, Hedgedog) régulant l'équilibre entre la prolifération et la différenciation des sébocytes. Le nombre de sébocytes ainsi générés et différenciés conditionne la taille et le volume de la glande sébacée formée et consécutivement la quantité de sébum synthétisée et délivrée à la surface de la peau.

Appliqué sur la peau, l'extrait selon l'invention limite la différenciation des sébocytes humains en sébocytes matures et réduit ainsi l'accumulation de lipides sébacés intracellulaires. En outre, il est capable de réduire l'expression de marqueurs de différenciation FADS2 et Mucin 1.

Le sébum est un mélange de substances huileuses (96%) et de sébocytes fragmentés. Sa composition est étonnamment complexe et comprend plusieurs centaines de composants dont 3 classes lipidiques :
- des triglycérides qui sont les constituants majeurs du sébum (60%) et qui possèdent des chaines d'acides gras uniques dans l'organisme (longueur de chaine à nombre impair et position des insaturations),
- des cires (25%) constituées d'esters d'acides gras et d'alcools gras à longues chaines,
- du squalène (15%) précurseur du cholestérol (2%).

Le squalène et les esters de cire sont des lipides spécifiques au sébum.

La peau grasse, qui affecte aussi bien les hommes que les femmes et apparaît avec la puberté puis ralentit jusque l'âge mûr, est généralement caractérisée par un taux de sébum supérieur à 150 µg/cm². Son écoulement ou taux d'excrétion est hautement dépendant de la température.

Appliqué sur la peau, l'extrait selon l'invention est capable de réduire le taux de sébum sécrété au niveau du visage pour tout type de peaux, en particulier pour des peaux caucasiennes et pour des peaux asiatiques.

Ainsi, en limitant la différenciation sébocytaire, l'extrait selon l'invention réduit la sécrétion de sébum de peaux grasses. En particulier, l'extrait selon l'invention permet :
- de réduire le taux de sébum sécrété
- de diminuer la taille des pores de manière immédiate et à long terme ;
- de réduire la brillance de la peau
- de diminuer le nombre d'imperfections sur la peau.

Sous l'effet de l'extrait de baies sans akènes de *Rosa canina* contenant des composés polyphénoliques la peau est donc matifiée et purifiée.

Afin d'illustrer ces effets cosmétiques sur la peau, les exemples suivants avec leurs résultats d'essais sont présentés.

### I. EXEMPLES

### I.1 Exemple 1 : Principe actif selon l'invention

Un exemple de procédé d'obtention d'un principe actif selon l'invention, comprend la mise en oeuvre des étapes suivantes :
- solubilisation de 50kg de poudre de baies de *Rosa canina* dans 100l d'eau et de butylène glycol (10l/90l), agitation pendant 2h,
- séparation des phases soluble et insoluble par filtration et élimination de la phase insoluble
- clarification par filtration de la phase soluble
- décoloration sur charbon actif
- filtration et filtration stérilisante sur filtre 0,22µ.

Le principe actif obtenu est un liquide limpide transparent présentant une teneur en matières sèches de 5,6g/l et un pH de 4,0.

Il contient 15mg/l de composés polyphénoliques, dont 0,27% en poids sont des composés polyhénoliques de type hydroxybenzoïques et des flavonoïdes.

### I.2 Exemple 2 : Principe actif selon l'invention

Un exemple de procédé d'obtention d'un principe actif selon l'invention, comprend la mise en oeuvre des étapes suivantes :
- solubilisation de 90kg de poudre de baies de *Rosa canina* dans 100l d'eau et de butylène glycol (10l/90l), agitation pendant 5h,
- séparation des phases soluble et insoluble par filtration et élimination de la phase insoluble
- clarification par filtration de la phase soluble
- décoloration au moyen d'un adjuvant PVPP
- filtration et filtration stérilisante sur filtre 0.22µ.

Le principe actif obtenu est un liquide limpide transparent présentant une teneur en matières sèches de 4,5g/l et un pH de 3,8.

Il contient 17mg/l de composés polyphénoliques, dont 0,38% en poids sont des composés polyhénoliques de type hydroxybenzoïques et des flavonoïdes.

### I.3 Exemple 3 : Utilisation d'un principe actif selon l'invention dans une crème visage

| | | | |
|---|---|---|---|
| A. | Eau | | qsp 100% |
| | Glycérine | | 7% |
| | Propylène glycol | | 1% |
| | | | |
| B | Myglyol 812N | (SASOL) | 7% |
| | Lanol 1688 | (SEPPIC) | 4% |
| | Dub 1632 | (STEARINERIE DUBOIS) | 0,5% |
| | Cutina MD | (BASF) | 2,5% |
| | Emulgade 1000NI | (BASF) | 1% |
| | Tween 60 | | 0,5% |
| | Stéarate d'isopropyle | (STEARINERIE DUBOIS) | 1% |
| | | | |
| C. | Sepisoft SP | (SEPPIC) | 1% |
| | | | |
| D. | Principe actif (exemple 1) | | 3% |
| | Conservateur | | 0,7% |

Les quantités indiquées sont données en pourcentage de poids.

Cette émulsion compacte, blanche, lisse, brillante, sans odeur, avec une texture souple et légère présente un pH de 5,6.

Elle présente une application aisée, sans sensation de gras, avec une pénétration rapide et un fini sec, doux et poudré.

Elle peut être obtenue par la mise en oeuvre des étapes suivantes :
- Mélanger A et chauffer au bain-marie à 80°C sous agitation magnétique,
- Mélanger B et chauffer au bain-marie à 80°C sous agitation magnétique,
- à 80°C, ajouter C dans A, puis additionner B sous rotor stator à 1800 tr/min,
- à 30°C, ajouter D, dans l'ordre indiqué, sous rotor stator à 1500 tr/min.

### I.4 Exemple 4 Utilisation d'un principe actif selon l'invention dans un lait visage

| | | | |
|---|---|---|---|
| A. | Eau | | qsp 100% |
| | Glycérine | | 2% |
| | Carbopol ultrez 10 | (LUBRIZOL) | 0,25% |
| | Xanthan Gum | (UNIPEX) | 0,20% |
| | | | |
| B. | Myglyol 812N | (SASOL) | 7% |
| | Tegesoft TN2 | (EVONIK) | 3% |
| | Dub 340 | (STEARINERIE DUBOIS) | 2% |
| | Palmitate d'isopropyle | (STEARINERIE DUBOIS) | 3% |
| | | | |
| C. | Principe actif (exemple 2) | | 3% |
| | Conservateur | | 0,7% |
| | | | |
| D | NaOH | | QSP pH 6.3 |

Les quantités indiquées sont données en pourcentage de poids.

Cette émulsion compacte, blanche, lisse, brillante, sans odeur, avec une texture souple et légère présente un pH de 6,3.

Elle présente une application aisée, sans sensation de gras, avec une pénétration rapide et un fini sec et doux.

Elle peut être obtenue par la mise en oeuvre des étapes suivantes :
- Mélanger A et chauffer au bain-marie à 80°C sous agitation magnétique, jusqu'à bonne dispersion du Carbopol Ultrez 10
- Mélanger B et chauffer au bain-marie à 80°C sous agitation magnétique,
- à 80°C, ajouter A dans B, sous rotor stator à 1800 tr/min,
- à 30°C, ajouter C puis D, dans l'ordre indiqué, sous rotor stator à 1500 tr/min.

### I.5 Exemple 5 Utilisation d'un principe actif selon l'invention dans une lotion nettoyante

| | | | |
|---|---|---|---|
| A. | Eau | | qsp 100% |
| | Glycérine | | 5% |
| | Propylène glycol | | 3% |
| | Urée | | 0,2% |
| | Ritabale 20 | (RITA) | 1% |
| | Oramix NS 10 | (SEPPIC) | 0,5% |
| | Tegosoft GM6 | (EVONIK) | 0,3% |
| | | | |
| B. | Principe actif (exemple 1) | | 3% |
| | Conservateur | | 0,7% |

Les quantités indiquées sont données en pourcentage de poids.

Cette lotion translucide présente un pH de 6,3.

Elle présente une application aisée, avec une pénétration rapide, sans coller, et un fini sec et doux.

Elle peut être obtenue par la mise en oeuvre des étapes suivantes :
- Mélanger A sous faible agitation magnétique,
- Ajouter A à B successivement.

### II. EVALUATION DE INEFFICACITE COSMETIQUE D'UN PRINCIPE ACTIF SELON L'INVENTION

### II. A. Identification de la fraction active de l'extrait

### a. Fractionnement du principe actif selon l'invention

Afin de déterminer la fraction active du principe actif selon l'invention (exemple 1), les espèces moléculaires majoritaires ont été fractionnées, à savoir les sucres (90% de l'actif) et les cendres (9% de l'actif).

### Purification des cendres : Fraction A

La fraction cendres est obtenue par ressolubilisation des résidus issus de l'incinération à 550°C dans un four à moufle électrique (VULCAN™ 3.550-NDI).

Le résidu est repris dans 5 mL d'eau distillée. La solution grisâtre obtenue est filtrée. L'analyse de la fraction A montre qu'elle contient 75% des cendres du principe actif.

### Purification des carbohydrates : fraction B

La composition et la quantité de sucres de la fraction B sont déterminées par chromatographie liquide ionique (système dionex ICS 3000).

La quantité de polyphénols résiduels est calculée par dosage colorimétrique.

La fraction B est purifiée en sucres et débarrassée des polyphénols : elle contient 91% des sucres du principe actif et une très faible quantité de polyphénols (<0,001 g/L soit 10% des polyphénols totaux).

### b. Etude de l'activité du principe actif selon l'invention et des fractions obtenues

L'objectif de cette étude est d'évaluer la capacité du principe actif (exemple 1) et de ses fractions A et B à diminuer la différenciation sébocytaire et la synthèse lipidique dans les cultures de sébocytes en 2D.

Cette étude a été réalisée sur des sébocytes humains normaux.

Le métabolisme de sébocytes en culture 2D a été évalué à l'aide d'une coloration Oil Red O permettant de visualiser les lipides neutres.

Le protocole est celui décrit au point D.1

Les résultats sont présentés dans le tableau 1 ci-après :

**Tableau 1 : Effet du principe actif et de ses fractions A et B sur la différenciation de sébocytes et la synthèse des lipides.**

| | Intensité de la coloration Oil red O (x10⁴ UA) | Synthèse des lipides (%) |
|---|---|---|
| Sébocytes non différenciés | | |
| Témoin | 984 ± 306 | |

| Sébocytes différenciés | | |
|---|---|---|
| Témoin | 4189 ± 258 | |
| Principe actif exemple 1 0,50% | 377 ± 136 | -91 |
| Fraction A 0,50% | 3909 ± 1281 | -7 |
| Fraction B 0,50% | 3806 ± 1066 | -9 |

Testé à 0,50% sur sébocytes humains normaux cultivés en 2D, le principe actif selon l'invention permet de limiter la différenciation sébocytaire et la synthèse des lipides de 91%.

La fraction A ne présente pas d'efficacité ce qui permet de montrer que les cendres ne confèrent pas d'efficacité au principe actif.

La fraction B possède une faible activité. Cette fraction est obtenue après ajout de charbon sur le principe actif selon l'invention et ne contient plus (ou en très faible quantité) les composés phénoliques de l'actif.

On peut donc en déduire que les composés phénoliques jouent un rôle important dans l'efficacité du principe actif selon l'invention, en association avec les autres composés de l'extrait.

### II. B. Tests in vivo sur peaux caucasiennes

### B.1- Etude de l'effet anti-séborrhée

### a) Mesure et visualisation de la sécrétion sébacée

L'objectif de cette étude a été de quantifier et visualiser, *in vivo,* sur volontaires, l'effet d'un extrait de Rosa canina selon l'invention (exemple 1) formulé à 2% en gel-émulsionné sur la production de sébum après délipidation, en comparaison à un groupe placebo.

Le gel émulsionné contenant l'extrait de l'exemple 1, utilisé pour cette étude présente la formule suivante :
- Cetearyl ethylhexanoate (Lanol 1688, Seppic) 5,0 %
- Behenyl alcohol / Arachidyl glucoside / Arachidyl alcohol (Montanonv 3,0% 202, Seppic)
- Principe actif de l'exemple 1 2,0%
- Polyacrylamide / C13-14 isoparaffin / Laureth 7 (Sepigel 305, Seppic) 2,0 %
- Conservateurs 0,7 %
- Cethyl alcool 0,5%
- Eau qsp 100%
Les volontaires sélectionnés pour cette étude ont été répartis en deux groupes de la façon suivante :
- Groupe A - placebo : groupe de 29 volontaires sains, de sexe féminin et masculin, d'âge compris entre 27 et 72 ans (âge moyen 45 ± 12 ans), ayant appliqué matin et soir une formule placebo en hémi-visage pendant 28 jours selon la randomisation établie. Les volontaires ont été sélectionnés selon les critères suivants :
   - un taux de sébum au niveau du front ≥ à 150 µg/cm²,
   - une surface des pores évaluée par projection de franges ≥ à 0,130 mm² au niveau des joues caractéristique d'un stade clinique 3 (sur une échelle de grade allant de 1 à 5).
- Groupe B - gel émulsionné contenant 2% du principe actif selon l'invention : groupe de 20 volontaires sains, de sexe féminin et masculin, d'âge compris entre 30 et 72 ans (âge moyen 47 ± 13 ans), ayant appliqué matin et soir l'actif selon l'invention en hémi-visage pendant 28 jours selon la randomisation établie. Les volontaires ont été sélectionnés selon les critères suivants :
   - un taux de sébum au niveau du front ≥ à 150 µg/cm²,
   - une surface des pores évaluée par projection de franges ≥ à 0,130 mm² au niveau des joues caractéristique d'un stade clinique 3 (sur une échelle de grade allant de 1 à 5).

L'effet anti-séborrhée des produits appliqués a été évalué après 28 jours de traitement biquotidien, en quantifiant et visualisant la production de sébum 1 heure après délipidation du front.

Les mesures ont été réalisées à l'aide d'un Sébumètre® SM 815 (Courage & Khazaka). Le Sébumètre® permet une mesure directe de la sécrétion sébacée.

Les résultats sont affichés sur l'écran en µg de sébum / cm² de peau.

La visualisation et la quantification du sébum sont réalisées au moyen d'un patch Sebutape® (SE100, Monaderm) appliqué au niveau du front.

Le Sebutape® est constitué d'une bandelette de polymère hydrophobe et lipophile découpée en logettes, particulièrement sensible aux sécrétions sébacées (sébum). La bandelette, après contact sur le front, est ensuite analysée à l'aide d'un logiciel d'analyse d'images QUANTISEB®, version 1.2.0.0 (Monaderm) pour quantifier le nombre et l'importance des taches dont la surface est proportionnelle à la quantité de lipides excrétés.

Le paramètre étudié est la surface totale occupée par les spots (mm²).

Le protocole opératoire de l'étude est le suivant :
- De J₋₁₄ à J₀ :
   - distribution d'une crème placebo à tous les volontaires.
   - applications biquotidiennes sur l'ensemble du visage.
- A J₋₁ : Nettoyage du visage à l'eau claire, sans appliquer de crème le soir.
- A J₀ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Délipidation du front.
      * Mesure du taux de sébum :
         - Mesure du taux de sébum 1h après délipidation (Sébumètre®).
         - Distribution des produits.
      * Mesure de la sécrétion sébacée :
         - Pose des Sébutape®
         - Retrait des Sébutape® 30 minutes après leurs poses.
         - Distribution des produits.
- Entre J₀ et J₂₇ : Applications biquotidiennes des produits.
- A J₂₇ : Nettoyage du visage à l'eau claire, sans appliquer de crème le soir.
- A J₂₈ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Délipidation du front.
      * Mesure du taux de sébum :
         - Mesure du taux de sébum 1h après délipidation (Sébumètre®).
         - Distribution des produits.
      * Mesure de la sécrétion sébacée :
         - Pose des Sébutape®
         - Retrait des Sébutape® 30 minutes après leurs poses.

Un résumé des résultats obtenus correspondant à l'effet du principe actif selon l'invention, formulé à 2% en gel-émulsionné et en comparaison au groupe placebo, sur le taux de sébum et sur la production de sébum après dilapidation, sont présentés dans le tableau 2 ci-après :

**Tableau 2 : Effet de l'extrait de Rosa canina selon l'invention, formulé à 2% en gel-émulsionné, sur le taux de sébum et la production de sébum mesuré après 28 jours d'applications biquotidiennes. Comparaison au groupe placebo.**

| | Variation / Placebo (%) |
|---|---|
| Taux de sébum | -14,9% |
| Production de sébum | -23,9% |

Ces résultats montrent que, dans les conditions de cette étude, après 28 jours de traitement biquotidien et en comparaison à un groupe placebo, l'extrait de *Rosa canina* selon l'invention formulé à 2% en gel-émulsionné :
- limite significativement la sécrétion de sébum en réduisant son taux produit en une heure au niveau du front de 14,9%,
- diminue significativement la surface totale occupée par les spots, caractéristique de la production de sébum.

Le principe actif selon l'invention permet donc de lutter contre la sécrétion excessive de sébum.

### b) Visualisation et quantification de la brillance de la peau

L'objectif de cette étude a été d'évaluer, *in vivo,* sur volontaires, l'effet d'un extrait de

Rosa canina selon l'invention (exemple 1) formulé à 2% en gel-émulsionné sur la brillance de la peau à l'aide de photographies numériques en comparaison à un placebo. L'étude a été réalisée sur 18 volontaires sains, de sexe féminin d'âge compris entre 27 et 51 ans (âge moyen 39±7 ans) présentant un taux de sébum au niveau du front ≥ à 150 µg/cm².

Des photographies numériques ont été réalisées avant et après 28 jours de traitement biquotidien du placebo ou du gel émulsionné contenant l'extrait de l'exemple 1.

Le gel émulsionné utilisé pour cette étude est le même que celui présenté au point B.1.a).

Les photographies permettant d'évaluer la brillance de la peau ont été prises dans des conditions d'éclairage reproductibles en utilisant le système Head Scan™ V04 (Orion TECHNOLAB). Le repositionnement des volontaires, aux différents temps de l'étude, est assuré par un système permettant de caler la tête autour des 3 axes de mouvement. De plus, la visualisation sur l'écran des photographies initiales (J0) assure le repositionnement du sujet.

Deux acquisitions de chaque côté du visage ont été réalisées en utilisant des filtres polarisant différents. En effet, les filtres polarisant positionnés sur les éclairages et sur le dispositif de prise de vue permettent de séparer l'information de brillance de la peau (composante spéculaire = polarisation parallèle), de celle due à la composante ambiante de la lumière, associée à une surface mate (composante non spéculaire = polarisation croisée). Ces deux images (spéculaires et non spéculaires) ont été utilisées pour étudier la composante spéculaire indépendamment de la composante ambiante selon la formule suivante : Composante spéculaire = Parallèle - Croisée

Le paramètre retenu pour cette étude est l'écart-type de la composante spéculaire représentant le niveau de brillance sur la zone d'intérêt découpée automatiquement au niveau du front.

Une diminution du paramètre écart-type est caractéristique d'une diminution de la brillance inesthétique de la peau.

Le protocole opératoire de l'étude est le suivant :
- De J₋₁₄ à J₀ :
   - distribution d'une crème placebo à tous les volontaires.
   - applications biquotidiennes sur l'ensemble du visage.
- A J₋₁ : Nettoyage du visage à l'eau claire, sans appliquer de crème le soir.
- A J₀ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Réalisation des photographies
   - Distribution des produits.
- Entre J₀ et J₂₇ : Applications biquotidiennes des produits.
- A J₂₇ : Nettoyage du visage à l'eau claire, sans appliquer de crème le soir.
- A J₂₈ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Réalisation des photographies.

Un résumé des résultats obtenus correspondant à l'effet du principe actif selon l'invention, formulé à 2% en gel-émulsionné est en comparaison au groupe placebo, sur la brillance de la peau évaluée sur photographies numériques, sont présentés dans le tableau 3 ci-après :

**Tableau 3: Effet de l'extrait de Rosa canina selon l'invention, formulé à 2% en gel-émulsionné, sur la brillance de la peau après 28 jours d'applications biquotidiennes. Comparaison au groupe placebo.**

| | Variation / Placebo (%) |
|---|---|
| Brillance de la peau | -5,6% |

Ces résultats montrent que dans les conditions de cette étude, après 28 jours de traitement biquotidien et en comparaison à un placebo, l'extrait de *Rosa canina* selon l'invention formulé à 2% en gel-émulsionné, permet une diminution significative de la brillance de la peau de 5,6%.

En réduisant la brillance, le principe actif selon l'invention se positionne comme un soin permettant de matifier la peau.

### B.2 - Effet sur l'apparence globale de la peau

### a) Etude de la surface des pores par projection de franges - Efficacité immédiate

L'objectif de cette étude a été d'évaluer, *in vivo,* sur volontaires, l'effet immédiat d'un extrait de Rosa canina selon l'invention (exemple 1) formulé à 2% en gel-émulsionné sur la surface des pores, à partir d'acquisitions réalisées par projection de franges et ce, en comparaison à un placebo.

Les mesures ont été réalisées avant et 15 minutes après application unique du gel émulsionné contenant l'extrait de l'exemple 1 ou du placebo au niveau des joues.

L'étude a été réalisée sur 20 volontaires sains, de sexe féminin et masculin, d'âge compris entre 27 et 71 ans (âge moyen 45 ± 13 ans) présentant des pores dilatés avec une surface des pores évaluée par projection de franges ≥ 0,130 mm² au niveau des joues, caractéristique d'un stade clinique de 3 (sur une échelle de grade allant de 1 à 5). Le gel émulsionné utilisé pour cette étude est le même que celui présenté au point B.1.a).

Les acquisitions ont été réalisées au niveau des joues à l'aide d'un appareil de projection de franges dédié spécifiquement à la mesure 3D du relief cutané (Eotech). Ce système (DermaTOP 1303, Breuckman) comprend un capteur de mesure associant un projecteur et une caméra CCD haute résolution relié à un logiciel d'acquisition Optocat (Eotech). Un système de repositionnement de la tête du volontaire selon les 3 axes de déplacement permet de retrouver la même zone de mesure aux différents temps de l'étude.

L'effet du produit a été mesuré sur une région d'intérêt de 10x10mm, découpée automatiquement sur l'acquisition originale. Une détection d'objets permet de segmenter les pores individuellement et d'en étudier différentes caractéristiques.

Le paramètre retenu pour cette étude est la taille moyenne des objets détectés, elle représente la surface moyenne des pores (en mm²) sur la zone d'intérêt.

Le protocole opératoire de l'étude est le suivant :
- A J₋₁ : Nettoyage du visage à l'eau claire, sans appliquer de crème le soir.
- A J₀ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Acquisition d'images (Projection de franges) au niveau des joues.
   - Application de 80µl de produits (actif selon l'invention en gel-émulsionné et placebo).
- A T_{15'} : Acquisition d'images (Projection de franges) au niveau des joues.

Un résumé des résultats obtenus correspondant à l'effet du principe actif selon l'invention, formulé à 2% en gel-émulsionné est en comparaison au groupe placebo, sur la surface des pores mesurée par projection de franges au niveau des joues, sont présentés dans le tableau 4 ci-après :

**Tableau 4: Effet de l'extrait de Rosa canina selon l'invention, formulé à 2% en gel-émulsionné, sur la surface des pores après 15 minutes. Comparaison au groupe placebo.**

| | Variation / Placebo (%) |
|---|---|
| T15 min | -9,4% |

Ces résultats montrent que, dans les conditions de cette étude, 15 minutes après une application unique et en comparaison au placebo, l'extrait de *Rosa canina* formulé à 2% en gel-émulsionné, diminue significativement la surface des pores de 9,4%.

Ainsi, l'extrait selon l'invention atténue instantanément l'apparence des pores

### b) Etude de la surface des pores par projection de franges - Efficacité à long terme

L'objectif de cette étude a été d'évaluer, *in vivo,* sur volontaires, l'effet d'un extrait de *Rosa canina* selon l'invention (exemple 1) formulé à 2% en gel-émulsionné sur la surface des pores évaluée au niveau des joues à partir d'acquisitions réalisées par projection de franges en comparaison à un groupe placebo.

Les volontaires sélectionnés ont été répartis en deux groupes de la façon suivante :
- Groupe A - placebo : tel que présenté au point B.1.a)
- Groupe B-extrait de *Rosa canina* : tel que présenté au point B.1.a)

La surface des pores au niveau des joues a été mesurée et quantifiée avant et après 28 jours de traitement biquotidien.

Le gel émulsionné utilisé pour cette étude est le même que celui présenté au point B.1.a).

Les acquisitions ont été réalisées au niveau des joues à l'aide d'un appareil de projection de franges dédié spécifiquement à la mesure 3D du relief cutané (Eotech). Ce système (DermaTOP 1303, Breuckman) comprend un capteur de mesure associant un projecteur et une caméra CCD haute résolution relié à un logiciel d'acquisition Optocat (Eotech). Un système de repositionnement de la tête du volontaire selon les 3 axes de déplacement permet de retrouver la même zone de mesure aux différents temps de l'étude.

L'effet du produit a été mesuré sur une région d'intérêt de 10x10mm, découpée automatiquement sur l'acquisition originale. Une détection d'objets permet de segmenter les pores individuellement et d'en étudier différentes caractéristiques.

Le paramètre retenu pour cette étude est la taille moyenne des objets détectés, elle représente la surface moyenne des pores (en mm²) sur la zone d'intérêt.

Le protocole opératoire de l'étude est le suivant :
- De J₋₁₄ à J₀ :
   - distribution d'une crème placebo à tous les volontaires.
   - applications biquotidiennes sur l'ensemble du visage.
- A J₋₁ : Nettoyage du visage à l'eau claire, sans appliquer de crème le soir.
- A J₀ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Acquisition d'images (Projection de franges) au niveau des joues.
- Entre J₀ et J₂₇ : Applications biquotidiennes des produits.
- A J₂₇ : Nettoyage du visage à l'eau claire, sans appliquer de crème le soir.
- A J₂₈ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Acquisition d'images (Projection de franges) au niveau des joues.

Un résumé des résultats obtenus correspondant à l'effet du principe actif selon l'invention, formulé à 2% en gel-émulsionné est en comparaison au groupe placebo, sur la surface des pores mesurée par projection de franges au niveau des joues, sont présentés dans le tableau 5 ci-après :

**Tableau 5: Effet de l'extrait de Rosa canina selon l'invention, formulé à 2% en gel-émulsionné, sur la surface des pores à long terme. Comparaison au groupe placebo.**

| | Variation / Placebo (%) |
|---|---|
| J28 | -8% |

Ces résultats montrent que, dans les conditions de cette étude, après 28 jours de traitement biquotidien et en comparaison à un placebo, l'extrait de *Rosa canina* selon l'invention formulé à 2% en gel-émulsionné, diminue significativement la surface des pores de 8,0%.

Ainsi, l'extrait selon l'invention améliore l'apparence et affine le grain de peau en réduisant la surface des pores.

### B.3 - Evaluation subjective

L'objectif de cette étude est d'évaluer et de quantifier les sensations ressenties par un groupe de volontaires ayant appliqué de façon biquotidienne un extrait de *Rosa canina* selon l'invention (exemple 1) formulé à 2% en gel-émulsionné à celles d'un groupe de volontaires ayant testé le placebo pendant 28 jours.

Les volontaires sélectionnés ont été répartis en deux groupes de la façon suivante :
- Groupe A- placebo : tel que présenté au point B.1.a)
- Groupe B-extrait de *Rosa canina* : tel que présenté au point B.1.a)

Le gel émulsionné utilisé pour cette étude est le même que celui présenté au point B.1.a).

Les sensations ressenties lors de l'utilisation de l'extrait de l'exemple 1 formulé à 2% en émulsion contre placebo pendant 28 jours d'applications biquotidiennes ont été évaluées à l'aide de questionnaires d'auto-évaluation.

Le questionnaire présentait des questions dites fermées. Les questions fermées étaient les suivantes :

**Tableau 6 : Questionnaire d'évaluation subjective de l'effet de l'extrait de Rosa canina selon l'invention.**

| | Pas d'accord | Plutôt pas d'accord | Plutôt d'accord | D'accord |
|---|---|---|---|---|
| Avec cette formule, ma peau paraît moins grasse. | | | | |
| Cette formule masque l'excès de sébum à la surface de ma peau. | | | | |
| Avec cette formule, ma peau brille moins. | | | | |
| Cette formule diminue les imperfections de ma peau. | | | | |
| Avec cette formule, mes pores semblent resserrés. | | | | |

Cette évaluation a eu lieu à la fin de l'étude.

Un résumé des résultats obtenus après analyse des questionnaires contenant les questions fermées auxquelles les panélistes de chaque groupe ont répondu à la fin de l'étude est présenté dans le tableau 7 ci-après :

**Tableau 7: Evaluation subjective de l'effet de l'extrait de Rosa canina selon l'invention, formulé à 2% en gel-émulsionné. Comparaison au groupe placebo.**

| | Cumul des réponses « d'accord » et « tout à fait d'accord » (%) | |
|---|---|---|
| | Placebo | Exemple 1 à 2% |
| Avec cette formule, ma peau paraît moins grasse. | 66% | 90% |
| Cette formule masque l'excès de sébum à la surface de ma peau. | 59% | 80% |
| Avec cette formule, ma peau brille moins. | 62% | 80% |
| Cette formule diminue les imperfections de ma peau. | 45% | 70% |
| Avec cette formule, mes pores semblent resserrés. | 52% | 80% |

Ces résultats montrent qu'après 28 jours d'applications biquotidiennes, l'extrait de *Rosa canina* selon l'invention formulé à 2% en gel-émulsionné, est perçu d'une manière générale comme plus efficace que le placebo.

En effet, pour plus de 80% des volontaires il limite les désagréments liés aux peaux grasses, tels que les pores dilatés, l'excès de sébum et la brillance à la surface de la peau.

### II. C. Tests in vivo sur peaux asiatiques : Etude de la capacité de l'extrait selon l'invention à limiter la sécrétion sébacée et l'apparition de lésions inflammées

L'objectif de l'étude est d'évaluer *in vivo* l'efficacité anti-séborrhée un extrait de *Rosa canina* selon l'invention (exemple 1) formulé à 3% en émulsion contre placebo au niveau du visage sur des personnes d'origine asiatique présentant une peau grasse.

Cette étude a été réalisée par le laboratoire d'ingénierie et de biologie cutanée du West China Hospital situé à Chengdu dans la province de Sichuan en Chine.

L'étude a été réalisée sur 20 volontaires sains, de sexe féminin et masculin, d'âge compris entre 18 et 30 ans, leur âge moyen étant de 23 ans ± 3 ans.

Le gel émulsionné contenant l'extrait de l'exemple 1, utilisé pour cette étude présente la formule suivante :
- Cetearyl ethylhexanoate (Lanol 1688, Seppic) 5,0 %
- Behenyl alcohol / Arachidyl glucoside / Arachidyl alcohol (Montanonv 3,0% 202, Seppic)
- Principe actif de l'exemple 1 3,0%
- Polyacrylamide / C13-14 isoparaffin / Laureth 7 (Sepigel 305, Seppic) 2,0 %
- Conservateurs 0,7 %
- Cethyl alcool 0,5%
- Eau qsp 100%
L'effet d'un extrait de *Rosa canina* selon l'invention a été évalué d'une part en mesurant le taux de sébum au niveau du front et d'autre part, par un comptage des lésions inflammées avant et après 28 jours de traitement biquotidien.

La mesure du taux de sébum est réalisée à l'aide d'un Sébumètre® SM 810 (Courage & Khazaka).

Le scorage clinique a été évalué selon l'évaluation ISGA (Investigator's Static Global Assessment).

Ce scorage est basé sur une échelle à 6 points allant de 0 (peau nette sans lésion) à 5 (lésions très sévères).

**Tableau 8 : Evaluation ISGA.**

| | |
|---|---|
| 0 | peau nette, sans lésion inflammatoire, une petite lésion inflammatoire maximum. |
| 1 | peau presque nette : rares lésions non-inflammatoires une petite lésion inflammatoire maximum. |
| 2 | plusieurs lésions non-inflammatoires avec quelques lésions inflammatoires maximum |
| 3 | beaucoup de lésions non- inflammatoires et inflammatoires une petite lésion nodulaire maximum |
| 4 | beaucoup de lésions non-inflammatoires et inflammatoires quelques petites lésions nodulaires maximum |
| 5 | beaucoup de lésions non-inflammatoires, quelques lésions nodulaires et des lésions kystiques potentielles. |

Le protocole opératoire de l'étude est le suivant :
- De J₋₁₄ à J₀ :
   - distribution d'une crème placebo à tous les volontaires.
   - applications biquotidiennes sur l'ensemble du visage.
- A J₋₁ : Nettoyage du visage à l'eau claire, sans appliquer de crème le soir.
- A J₀ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Mesure du taux de sébum (Sébumètre®).
   - Mesure du nombre de lésions.
- Entre J₀ et J₂₇ : Applications biquotidiennes des produits.
- A J₂₇ : Nettoyage du visage à l'eau claire, sans appliquer de crème le soir.
- A J₂₈ :
   - Les volontaires viennent au laboratoire sans avoir appliqué de produit sur le visage depuis la veille (ni eau, ni crème, ni maquillage).
   - Phase d'acclimatation de 15 minutes dans une salle contrôlée en température et hygrométrie.
   - Mesure du taux de sébum (Sébumètre®).
   - Mesure du nombre de lésions.

Un résumé des résultats est présenté dans les tableaux 9 et 10 ci-après.

**Tableau 9 : Effet anti-séborrhée de l'extrait de Rosa canina selon l'invention formulé à 3% en comparaison au placebo.**

| | Variation / Placebo (%) |
|---|---|
| Effet anti-séborrhée | -14,8% |

**Tableau 10 : Effet d'un extrait de Rosa canina selon l'invention formulé à 3% sur le nombre de lésions en comparaison au placebo.**

| | Variation / Placebo (%) |
|---|---|
| Nombre de boutons | -20,9% |
| Nombre de comédons | +1,8% |

Dans les conditions de cette étude, après 28 jours de traitement biquotidien et en comparaison à un placebo, l'extrait de *Rosa canina* selon l'invention formulé à 3% en gel-émulsionné, réduit significativement le taux de sébum au niveau du front de 14,8%. De plus, après utilisation de l'extrait de *Rosa canina* selon l'invention formulé à 3% pendant 28 jours, un comptage des lésions a permis de mettre en évidence une diminution significative du nombre de boutons de 20,9%.

Ainsi, l'extrait de *Rosa canina* selon l'invention permet d'améliorer les désagréments esthétiques liés aux problèmes de peau grasse de volontaires asiatiques.

### II. D. Effet de l'extrait selon l'invention sur la différenciation sébocytaire et la lipogenèse

### D.1- Etude sur modèle 2D de sébocytes humains normaux

L'objectif de cette étude est d'évaluer l'effet de l'extrait de *Rosa canina* selon l'invention (exemple 1) sur sa capacité à limiter la différenciation sébocytaire et la lipogenèse dans les cultures de sébocytes en 2D.

Cette étude a été réalisée sur des sébocytes humains.

Le métabolisme de sébocytes en culture 2D a été évalué à l'aide d'une coloration Oil Red O permettant de visualiser le taux de lipides.

Le protocole opératoire de l'étude est décrit en suivant.

A J0, les sébocytes sont ensemencés avec un milieu de croissance adéquat.

A J3, le milieu de croissance est remplacé par du milieu de différentiation adéquat contenant ou non un extrait selon l'exemple 1 à 0,25% et 0,50% (V/V).

Les milieux de culture sont changés tous les 2 jours.

A J7, on effectue une coloration Oil Red O :
- Fixer les cellules avec une solution de formaldéhyde à 10%
- Rincer les cellules
- Colorer les cellules à l'aide d'une solution d'Oil Red O
- Rincer les cellules

La visualisation des gouttelettes lipidiques est réalisée sur un microscope IX 70 (Olympus) couplé à un système d'analyse d'images (logiciel NIS-Elements AR-Nikon).

De plus, une analyse quantitative des images a été réalisée à l'aide du logiciel Matlab®, version R2012 (The Matworks).

Les résultats sont résumés dans le tableau 11 ci-après.

**Tableau 11 : Effet de l'extrait de Rosa canina selon l'invention sur la différenciation sébocytaire et la synthèse lipidique de sébocytes humains normaux cultivés en 2D.**

| | **Intensité de la coloration Oil red O (x 10⁴ UA)** | **Synthèse des lipides (%)** |
|---|---|---|
| **Sébocytes non différenciés** | | |
| Témoin | 984 ± 306 | |

| **Sébocytes différenciés** | | |
|---|---|---|
| Témoin | 4189 ± 258 | |
| Exemple 1 à 0,10% | 2375 ± 270 | -43 |
| Exemple 1 à 0,50% | 377 ± 136 | -91 |

On constate que testé à 0,50% sur sébocytes humains normaux cultivés en 2D, un extrait de *Rosa canina* selon l'invention permet de limiter la synthèse des lipides de 91%.

### D.2 - Etude sur modèle 3D de sébocytes

L'objectif de cette étude est d'évaluer l'effet d'un extrait de *Rosa canina* selon l'invention (exemple 1) sur sa capacité à diminuer la différenciation sébocytaire et la lipogenèse dans un modèle 3D de sébocytes.

Le métabolisme de sébocytes a été évalué à l'aide :
- de l'étude par PCR quantitative de l'expression de FADS2 (fatty acid desaturase 2), enzyme responsable de la production des lipides caractéristiques du sébum et de Mucin 1, marqueur de la différenciation terminale sébocytaire
- de la coloration Oil Red O permettant de visualiser le taux de lipides.

Le protocole opératoire est décrit en suivant.

A J0, les sébocytes SEB0662 (sébocytes humains issus d'une lignée) sont ensemencés sur des inserts.

A J2, les cultures sont placés à l'interface air liquide en milieu de différenciation adéquat contenant ou non un extrait de Rosa canina à 0,50% (V/V).

Les milieux de culture sont changés tous les 2 jours.

AJ16:
- Etude par PCR quantitative
   Les modèles 3D sont récupérés et les ARN totaux extraits.
   Les ARN ont été reverse-transcripts et les ADN complémentaires obtenus ont été analysés par la technique de PCR quantitative.
   Les ARNm de Mucin 1 et de fatty acid desaturase 2 (FADS2) sont analysés. Parallèlement, les ARNm de la glyceraldehyde-3-phosphate dehydrogenase (GAPDH), témoin interne de référence, ont également été mesurés.
   La quantification de l'incorporation de fluorescence (SYBR Green) est mesurée en continu à l'aide d'un thermocycleur (LightCycler LC480 - ROCHE). L'analyse des Ct (quantification relative) est réalisée à l'aide du logiciel LC480 (ROCHE).
- Coloration Oil Red O
   A J16, les modèles 3D sont récupérés et congelés. Des coupes (10 µm) sont ensuite réalisées à l'aide d'un cryostat.
   Les coupes sont :
   - fixées au formol 4% pendant 10 min
   - rincées en eau et en isopropanol 60%
   - colorées à l'aide d'une solution d'Oil Red O pendant 15 min
   - rincées en isopropanol 60%
   - colorées à l'aide d'une solution d'hématoxyline

Les résultats sont résumés dans le tableau 12 ci-après pour l'étude de l'expression de FADS2 et de Mucin 1 par PCR quantitative, et dans le tableau 13 ci-après pour la visualisation des lipides par coloration Oil Red O.

**Tableau 12 : Effet de l'extrait de Rosa canina selon l'invention sur l'expression de FADS2 et Mucin 1.**

| | Expression (%) | |
|---|---|---|
| | FADS2 | Mucin 1 |
| Témoin | 100 ± 0 | 100 ± 0 |
| Exemple 1 à 0,50% | 78 ± 9 | 57 ± 9 |

**Tableau 13 : Effet de l'extrait de Rosa canina selon l'invention sur la synthèse lipidique dans un modèle 3D de sébocytes.**

| | Surface colorée Oil Red O (µm²) | Synthèse des lipides (%) |
|---|---|---|
| Témoin | 6611 | |
| Exemple 1 à 0,50% | 5213 | -21% |

Ces résultats montrent que, testé à 0,50% sur sébocytes cultivés en 3D, un extrait de *Rosa canina* selon l'invention permet :
- de réduire respectivement l'expression des marqueurs de différenciation FADS2 et Mucin 1 de 22 et 43%, et
- de limiter significativement la synthèse des lipides de 21%.

Ces différents résultats montrent bien l'efficacité anti-séborrhée d'un extrait hydroglycolique de baies sans akènes de *Rosa canina* contenant des composés polyphénoliques.

## Revendications

1. Utilisation cosmétique non-thérapeutique comme actif anti-séborrhée d'un extrait hydroglycolique de baies sans akènes de *Rosa canina* contenant des composés polyphénoliques.

2. Utilisation cosmétique non-thérapeutique selon la revendication 1, pour lutter contre la sécrétion excessive de sébum et les manifestations cutanées associées.

3. Utilisation cosmétique non-thérapeutique selon l'une des précédentes revendications, pour limiter la brillance et les imperfections de la peau liées à la sécrétion de sébum.

4. Utilisation cosmétique non-thérapeutique selon l'une des précédentes revendications, pour réduire la surface des pores de la peau.

5. Utilisation cosmétique non-thérapeutique selon l'une des précédentes revendications pour diminuer la sécrétion de sébum.

6. Utilisation cosmétique non-thérapeutique selon l'une des précédentes revendications, **caractérisée en ce que** l'extrait est présent dans une composition comprenant un milieu cosmétiquement acceptable.

7. Utilisation cosmétique non-thérapeutique selon la revendication 5, **caractérisée en ce que** l'extrait est présent dans la composition à raison de 0,5 à 10% en poids de matières sèches.

8. Utilisation cosmétique non-thérapeutique selon l'une des précédentes revendications, **caractérisée en ce que** les composés polyphénoliques comprennent des flavonoïdes et des composés hydroxybenzoïques.

9. Utilisation cosmétique non-thérapeutique selon l'une des précédentes revendications, **caractérisée** ce que l'extrait est obtenu à l'aide d'un solvant constitué par un mélange d'eau et de butylène glycol.

10. Procédé cosmétique non-thérapeutique de soin de la peau pour lutter contre la sécrétion excessive de sébum et les manifestations cutanées associées, **caractérisé en ce qu'**il consiste à appliquer sur la peau une composition cosmétique comprenant un extrait hydroglycolique de baies sans akènes de *Rosa canina* contenant des composés polyphénoliques.

## Patentansprüche

1. Nichttherapeutische, kosmetische Verwendung eines hydroglykolischen Extrakts der Beeren von *Rosa canina* ohne Achäne als Mittel gegen Seborrhö, das Polyphenolverbindungen enthält.

2. Nichttherapeutische, kosmetische Verwendung nach Anspruch 1, zur Bekämpfung von übermäßigen Talgausscheidungen und von damit verbundenen Hauterscheinungen.

3. Nichttherapeutische, kosmetische Verwendung nach einem der vorhergehenden Ansprüche, um den Glanz und die mit der Ausscheidung von Talg verbundenen Mängel der Haut zu begrenzen.

4. Nichttherapeutische, kosmetische Verwendung nach einem der vorhergehenden Ansprüche, um die Oberfläche der Hautporen zu verringern.

5. Nichttherapeutische, kosmetische Verwendung nach einem der vorhergehenden Ansprüche, um die Talgausscheidung zu verringern.

6. Nichttherapeutische, kosmetische Verwendung nach einem der vorhergehenden Ansprüche, **dadurch gekennzeichnet, dass** der Extrakt in einer Zusammensetzung vorliegt, die eine kosmetisch akzeptierbare Umgebung umfasst.

7. Nichttherapeutische, kosmetische Verwendung nach Anspruch 5, **dadurch gekennzeichnet, dass** der Extrakt in der Trockenmasse mit 0,5 bis 10 Gew.-% der Zusammensetzung vorliegt.

8. Nichttherapeutische, kosmetische Verwendung nach einem der vorhergehenden Ansprüche, **dadurch gekennzeichnet, dass** die Polyphenolverbindungen Flavonoide und Hydroxybenzoeverbindungen aufweisen.

9. Nichttherapeutische, kosmetische Verwendungen nach einem der vorhergehenden Ansprüche, **dadurch gekennzeichnet, dass** der Extrakt mithilfe eines Lösungsmittels erhalten wird, das durch eine Mischung von Wasser und Butylenglykol gebildet ist.

10. Nichttherapeutisches, kosmetisches Verfahren zur Hautpflege, um die übermäßige Talgausscheidung und die damit verbundenen Hauterscheinungen zu bekämpfen, **dadurch gekennzeichnet, dass** dieses darin besteht, auf die Haut eine kosmetische Zusammensetzung aufzutragen, die einen hydroglykolischen Extrakt der Beeren von *Rosa canina* ohne Achäne mit Polyphenolverbindungen aufweist.

## Claims

1. Non-therapeutic cosmetic use as an anti-seborrheic active agent of a hydroglycolic extract of achene-free *Rosa canina* hips containing polyphenolic compounds.

2. Non-therapeutic cosmetic use according to claim 1, for combatting excessive secretion of sebum and associated skin manifestations.

3. Non-therapeutic cosmetic use according to either of the preceding claims, for limiting shine and imperfections of the skin related to the secretion of sebum.

4. Non-therapeutic cosmetic use according to any of the preceding claims, for reducing the surface area of the skin pores.

5. Non-therapeutic cosmetic use according to any of the preceding claims, for reducing sebum secretion.

6. Non-therapeutic cosmetic use according to any of the preceding claims, **characterised in that** the extract is present in a composition comprising a cosmetically acceptable medium.

7. Non-therapeutic cosmetic use according to claim 5, **characterised in that** the extract is present in the composition at 0.5% to 10% by weight of dry matter.

8. Non-therapeutic cosmetic use according to any of the preceding claims, **characterised in that** the polyphenolic compounds comprise flavonoids and hydroxybenzoic compounds.

9. Non-therapeutic cosmetic use according to any of the preceding claims, **characterised in that** the extract is obtained by means of a solvent consisting of a mixture of water and butylene glycol.

10. A non-therapeutic cosmetic skincare method for combatting excessive secretion of sebum and associated skin manifestations, **characterised in that** it comprises applying to the skin a cosmetic composition comprising a hydroglycolic extract of achene-free *Rosa canina* hips containing polyphenolic compounds.
